# EUROPEAN PATENT APPLICATION

(11) **EP 2 848 184 A1**
(43) Date of publication of application: **18.03.2015**
(21) Application number: 13787082.0
(22) Date of filing: 01.05.2013
(51) Int. Cl.: A61B 1/00

(54) **MAGNETIC FIELD GENERATION DEVICE, AND CAPSULE-TYPE MEDICAL DEVICE GUIDE SYSTEM**

(30) Priority: 07.05.2012 JP 2012106331
(71) Applicant: Olympus Medical Systems Corp., Tokyo 151-0072 (JP)
(72) Inventor: CHIBA, Atsushi, Tokyo 151-0072 (JP); KAWANO, Hironao, Tokyo 151-0072 (JP)
(74) Representative: Schicker, Silvia
(86) International application number: PCT/JP2013/062727
(87) International publication number: WO 2013/168659

(57) **Abstract**

Provide are a small magnetic field generation apparatus, in a guiding apparatus guiding the capsule medical device introduced in a subject, generating a magnetic field for guiding the capsule medical device by a permanent magnet and being capable of shielding the magnetic field at a desired timing, and a capsule medical device guiding system including the magnetic field generation apparatus. The guiding apparatus 20 as the magnetic field generation unit includes a plurality of permanent magnets 25a and 25b generating a magnetic field, and a magnetic field state change driving unit 252 changing relative relationship of the permanent magnets 25a and 25b to switch between a magnetic field generation state in which the permanent magnets 25a and 25b generate the guiding-magnetic field MF in an effective magnetic field region 100 and a magnetic field shielded state in which the permanent magnets 25a and 25b do not generate the guiding-magnetic field MF in the effective magnetic field region 100.

## Description

### Field

The present invention relates to a magnetic field generation apparatus and a capsule medical device guiding system for generating magnetic field for magnetically guiding a capsule medical device introduced into a subject. Background

In the past, in the field of endoscopes, a capsule-type endoscope formed to have such size that can be introduced into a digestive tract of a subject such as a patient has been developed. The capsule-type endoscope has an image-capturing function and a wireless communication function inside of a capsule-shaped casing, and after the capsule-type endoscope is swallowed from the mouth of the subject, the capsule-type endoscope moves within the digestive tract due to peristaltic movement and the like, during which the digestive tract successively obtains image data of images inside of the internal organs of the subject (hereinafter also referred to as in-vivo images), and wirelessly transmits the image data to a reception apparatus provided outside of the subject. The image data received by the reception apparatus are retrieved into an image display apparatus and are subjected to predetermined image processing. Accordingly, the in-vivo images are displayed on a display as a still picture display or a motion picture display. A user such as a doctor or a nurse observes an in-vivo image displayed on the image display apparatus as described above to diagnoses the state of the internal organs of the subject.

In recent years, a guiding system for guiding a capsule-type endoscope introduced into a subject using magnetic force (which will be hereinafter referred to as magnetic guidance) has been suggested (for example, see Patent Literature 1 and Patent Literature 2). In general, in such guiding system, a permanent magnet is provided inside of the capsule-type endoscope, and a guiding apparatus that has a magnetic field generation unit such as an electromagnet is provided outside of the subject. The magnetic field that is generated by the magnetic field generation unit is applied to the permanent magnet provided inside of the capsule-type endoscope, and the capsule-type endoscope is magnetically guided to a desired position by magnetic attraction occurred from the magnetic field.

The guiding apparatus may include a display unit for receiving image data obtained by the capsule-type endoscope and displaying the in-vivo image, an input device for operating the position, the posture of the capsule-type endoscope, and the like. In the case of the guiding apparatus, a user can refer to the in-vivo image displayed on the display unit, and use the input device to operate the magnetic guidance of the capsule-type endoscope.

A system has been developed, in which the magnetic field is applied to the capsule-type endoscope outside of the subject, so that the system performs signal control such as ON/OFF of the switch of the capsule-type endoscope (for example, see Patent Literature 3).

### Citation List

### Patent Literature

Patent Literature 1: Japanese Laid-open Patent Publication No. 2006-68501
Patent Literature 2: Japanese National Publication of International Patent Application No. 2008-503310
Patent Literature 3: International Publication WO 2007/083708

### Summary

### Technical Problem

By the way, in case of using a permanent magnet as the magnetic field generation generating a magnetic field for guiding a capsule medical device, the generation of the magnetic field by the permanent magnet cannot be turn on/off unlike the electromagnet. That is, because the permanent magnet always generates a magnetic field with a predetermined strength, it is necessary to lower the strength of the magnetic field or preferably to shield the magnetic field for preventing unintended movement of the capsule medical device and influence on a subject when a guiding operation of the capsule medical device is not performed. For that point, Patent Literature 3 discloses that a permanent magnet is covered by a covering unit formed of ferromagnetic material when a magnetic field generation unit is not used. However, in this case, a size and a weight of the magnetic field generation unit become larger and heavier.

The present invention has been made considering the above and an object thereof is to provide a small magnetic field generation apparatus, in a guiding apparatus guiding the capsule medical device introduced in a subject, generating a magnetic field for guiding the capsule medical device by a permanent magnet and being capable of shielding the magnetic field at a desired timing, and to provide a capsule medical device guiding system including the magnetic field generation apparatus.

### Solution to Problem

To solve the above-described problem and achieve the object, a magnetic field generation apparatus according to the present invention generates a guiding-magnetic field which is a magnetic field for magnetically guiding a target object and includes a plurality of permanent magnets configured to generate a magnetic field, and a switching unit configured to change relative relationship of the plurality of permanent magnets to switch between a first state in which the plurality of permanent magnets generate the guiding-magnetic field in a predetermined region and a second state in which the plurality of permanent magnets do not generate the guiding-magnetic field in the region.

In the above-described magnetic field generation apparatus, in the first state, the plurality of permanent magnets form open magnetic circuit, and in the second state, the plurality of permanent magnets form closed magnetic circuit.

In the above-described magnetic field generation apparatus, the plurality of permanent magnets are arranged such that magnetization directions are parallel to each other and different poles are arranged to face each other, wherein, in the first state, the plurality of permanent magnets are spaced apart, and in the second state, the plurality of permanent magnets are close to each other.

In the above-described magnetic field generation apparatus, the plurality of permanent magnets are arranged such that magnetization directions are parallel to each other, wherein, in the first state, the plurality of permanent magnets are arranged such that same poles are arranged to face each other, and in the second state, the plurality of permanent magnets are arranged such that poles different from each other are arranged to face each other.

In the above-described magnetic field generation apparatus, in the second state, the plurality of permanent magnets are configured such that pole surfaces different from each other are further brought in proximity to each other.

In the above-described magnetic field generation apparatus, the plurality of permanent magnets arranged to be spaced apart from each other such that magnetization directions are parallel to each other and different poles are arranged to face each other, the magnetic field generation apparatus further comprises a magnetic shield member made of a ferromagnetic material, wherein, in the first state, the magnetic shield member is in contract with the pole surfaces of the permanent magnets, and in the second state, the magnetic shield member is spaced apart from the pole surface.

The above-described magnetic field generation apparatus further includes a magnetic field state identifying unit configured to identify the first state and the second state, and a notifying unit configured to notify an identifying result given by the magnetic field state identifying unit.

In the above-described magnetic field generation apparatus, the magnetic field state identifying unit detects the magnetic field generated by the magnetic field generation apparatus.

In the above-described magnetic field generation apparatus, the magnetic field state identifying unit detects force between the plurality of permanent magnets.

In the above-described magnetic field generation apparatus, the notifying unit notifies the identifying result with visual information.

In the above-described magnetic field generation apparatus, the notifying unit notifies the identifying result with audio information.

In the above-described magnetic field generation apparatus, the target object is a capsule medical device including a magnet and introduced into a subject.

A capsule medical device guiding system according to the present invention includes: a magnetic field generation apparatus for generating a guiding-magnetic field which is a magnetic field for magnetically guiding a target object, wherein the magnetic field generation apparatus includes a plurality of permanent magnets configured to generate a magnetic field, and a switching unit configured to change relative relationship of the plurality of permanent magnets to switch between a first state in which the plurality of permanent magnets generate the guiding-magnetic field in a predetermined region and a second state in which the plurality of permanent magnets do not generate the guiding-magnetic field in the region; and a capsule medical device including a magnet and introduced into a subject, wherein the capsule medical device is magnetically guided by the guiding-magnetic field in the predetermined region.

The above-described capsule medical device guiding system further includes a magnetic field state identifying unit configured to identify the first state and the second state, and a control unit configured to control operation of the switching unit in accordance with an identifying result given by the magnetic field state identifying unit and a status of inspection with the capsule medical device.

### Advantageous Effects of Invention

According to the present invention, in the magnetic field generation apparatus using the permanent magnets to generate the guiding-magnetic field for guiding the capsule medical device introduced into the subject, the relative relationship of a plurality of permanent magnets is changed with the switching unit to switch the first state in which the plurality of permanent magnets generate the guiding-magnetic field in the predetermined region and the second state in which the plurality of permanent magnets do not generate the guiding-magnetic field in the region, and therefore, the small magnetic field generation apparatus capable of shielding the magnetic field at a desired timing can be realized, and further, the capsule medical device guiding system having the magnetic field generation apparatus can be realized.

### Brief Description of Drawings

FIG. 1 is a diagram illustrating an example of configuration of capsule medical device guiding system according to a first embodiment of the present invention.
FIG. 2 is a schematic view illustrating a schematic configuration of guiding apparatus as shown in FIG. 1.
FIG. 3 is a cross sectional schematic view illustrating an example of an internal structure of the capsule-type endoscope as shown in FIG. 1.
FIG. 4 is a schematic view illustrating configuration and operation principle of a magnetic field generation unit as shown in FIG. 1.
FIG. 5 is a schematic view illustrating configuration and operation principle of a magnetic field generation unit as shown in FIG. 1.
FIG. 6 is a schematic view for explaining operation of a flat plane position change unit, a vertical position change unit, an elevation angle change unit, and a traverse angle change unit as shown in FIG. 1.
FIG. 7 is a flowchart illustrating operation of a capsule medical device guiding system as shown in FIG. 1.
FIG. 8 is a partial cross-sectional side surface view schematically illustrating the guiding apparatus in magnetic field shielded state.
FIG. 9 is a partial cross-sectional side surface view schematically illustrating the guiding apparatus in a case of weak magnetic field generation state.
FIG. 10 is a partial cross-sectional side surface view schematically illustrating a guiding apparatus in a case of magnetic field generation state.
FIG. 11A is a schematic view illustrating a configuration of magnetic field generation unit (magnetic field generation state) provided in a guiding apparatus of a second embodiment of the present invention.
FIG. 11B is a schematic view illustrating a configuration of magnetic field generation unit (magnetic field shielded state) provided in the guiding apparatus of the second embodiment of the present invention.
FIG. 12A is a schematic view illustrating a configuration of magnetic field generation unit (magnetic field generation state) provided in the guiding apparatus of a third embodiment of the present invention.
FIG. 12B is a schematic view illustrating a configuration of magnetic field generation unit (magnetic field shielded state) provided in the guiding apparatus of the third embodiment of the present invention.
FIG. 13 is a schematic view illustrating a configuration of magnetic field generation unit (magnetic field shielded state) provided in a guiding apparatus according to modification 3-1 of the third embodiment of the present invention.
FIG. 14A is a schematic view illustrating a configuration of magnetic field generation unit (magnetic field generation state) provided in a guiding apparatus according to a fourth embodiment of the present invention.
FIG. 14B is a schematic view illustrating a configuration of magnetic field generation unit (magnetic field shielded state) provided in the guiding apparatus according to the fourth embodiment of the present invention.
FIG. 15 is a schematic view illustrating a schematic configuration of guiding apparatus according to a fifth embodiment of the present invention.
FIG. 16 is a schematic view for explaining operation state of the guiding apparatus as shown in FIG. 15. Description of Embodiments

Hereinafter, a magnetic field generation apparatus and a capsule medical device guiding system according to embodiments of the present invention will be explained with reference to drawings. In the explanation below, a guiding system for a capsule-type endoscope using a capsule-type endoscope serving as a capsule medical device introduced from the mouth of the subject into the subject and drifts about in a liquid accumulated in the stomach of a subject is used for example, but the present invention is not limited by the embodiments. More specifically, the present invention can be applied to systems for guiding various kinds of capsule medical devices, for example, a capsule-type endoscope that moves through the lumens from the esophagus to the anus of the subject, and a capsule-type endoscope introduced together with isotonic solution from the anus. In the explanation below, each drawing is nothing but schematically illustrates the relationship of the shape, the size, and the position in such a manner that the contents of the present invention can be understood. Therefore, the present invention is not limited to the relationship of the shape, the size, and the position as illustrated in each drawing, for example.

In the explanation below, for example, magnetic field generation apparatus according to the present invention is applied to a guiding apparatus generating magnetic field for magnetically guiding the capsule medical device (capsule-type endoscope) in the capsule medical device guiding system. The magnetic field generation apparatus of the present invention can also be applied to those other than the capsule medical device guiding system. For example, the magnetic field generation apparatus of the present invention may be applied to a guiding apparatus for magnetically guiding a target object other than a capsule-type endoscope.

In the present application, magnetic field for magnetically guiding a target object such as a capsule medical device may also be referred to as guiding-magnetic field.

It should be noted that, in the description of the drawings, the same portions are denoted with the same reference numerals.

### (First embodiment)

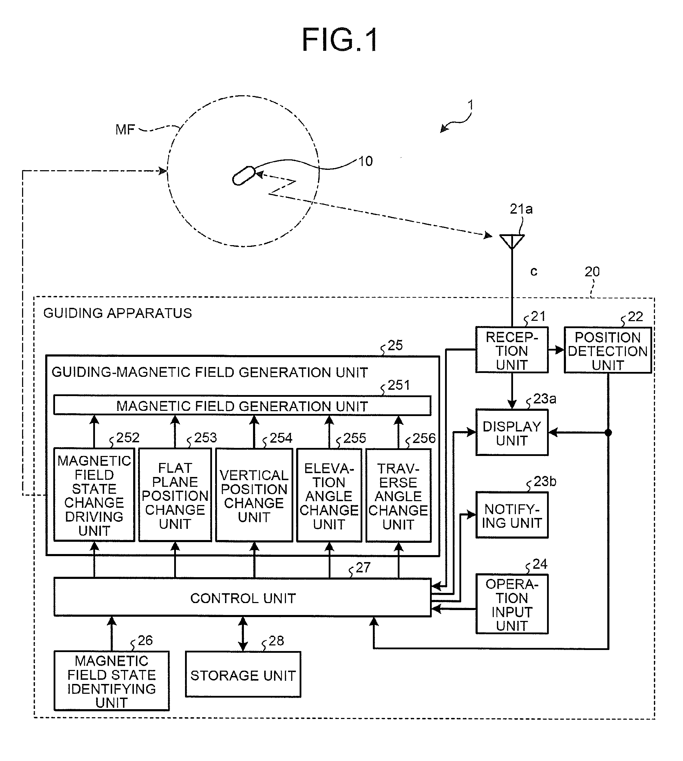

FIG. 1 is a schematic diagram illustrating an example of configuration of capsule medical device guiding system according to the first embodiment of the present invention. As illustrated in FIG. 1, the capsule medical device guiding system according to the first embodiment (which may be hereinafter simply referred to as guiding system) 1 is a capsule medical device that is introduced into the body cavity of a subject, and includes a capsule-type endoscope 10 provided with a permanent magnet therein and a guiding-magnetic field generation unit 25 for generating three-dimensional magnetic field MF, and also includes a guiding apparatus 20 serving as a magnetic field generation apparatus for magnetically guiding the capsule-type endoscope 10 introduced into the subject by this magnetic field MF.

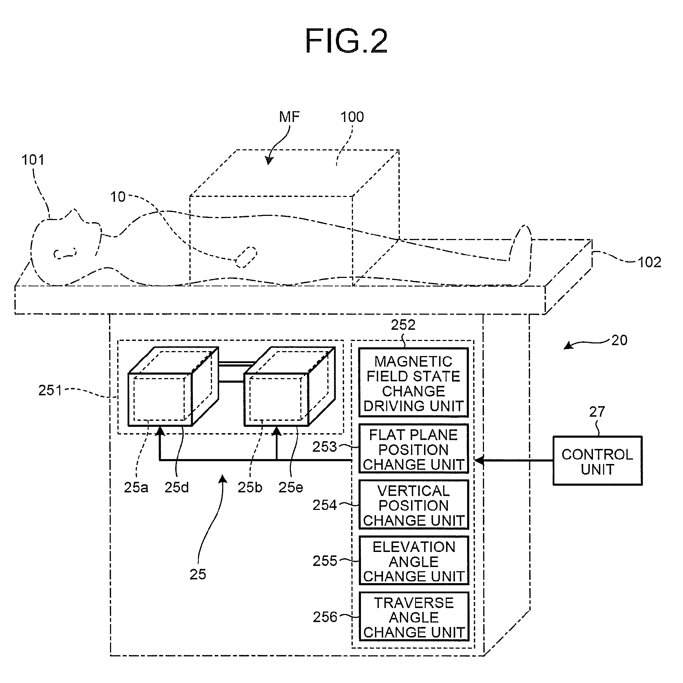

FIG. 2 is a view schematically illustrating capsule-type endoscope inspection that is performed with the guiding system 1. The capsule-type endoscope inspection is done while, for example, a subject 101 lies on a bed (subject bed) 102 under which a guiding-magnetic field generation unit 25 is provided. The guiding-magnetic field generation unit 25 generates the magnetic field MF of which intensity is high enough to guide the capsule-type endoscope in a region 100 including an inspection target portion (stomach, small intestine, large intestine, and the like) of the subject 101. Hereinafter, the region 100 in which the magnetic field MF capable of guiding the capsule-type endoscope 10 is generated will be referred to as an effective magnetic field region.

The capsule-type endoscope 10 is introduced into the subject 101 by ingestion and the like together with predetermined liquid (for example, water), and thereafter, the capsule-type endoscope 10 moves inside of the digestive tract, and ultimately, the capsule-type endoscope 10 is discharged out of the subject 101. During this period, the capsule-type endoscope 10 drifts about in the liquid introduced into the inside of an internal organ of the subject 101 (for example, inside of the stomach), and the capsule-type endoscope 10 successively captures images in the subject 101 while the capsule-type endoscope 10 is magnetically guided by the magnetic field generated by the guiding apparatus 20, and the capsule-type endoscope 10 successively, wirelessly transmits image information (image data) corresponding to the in-vivo images obtained by image-capturing process.

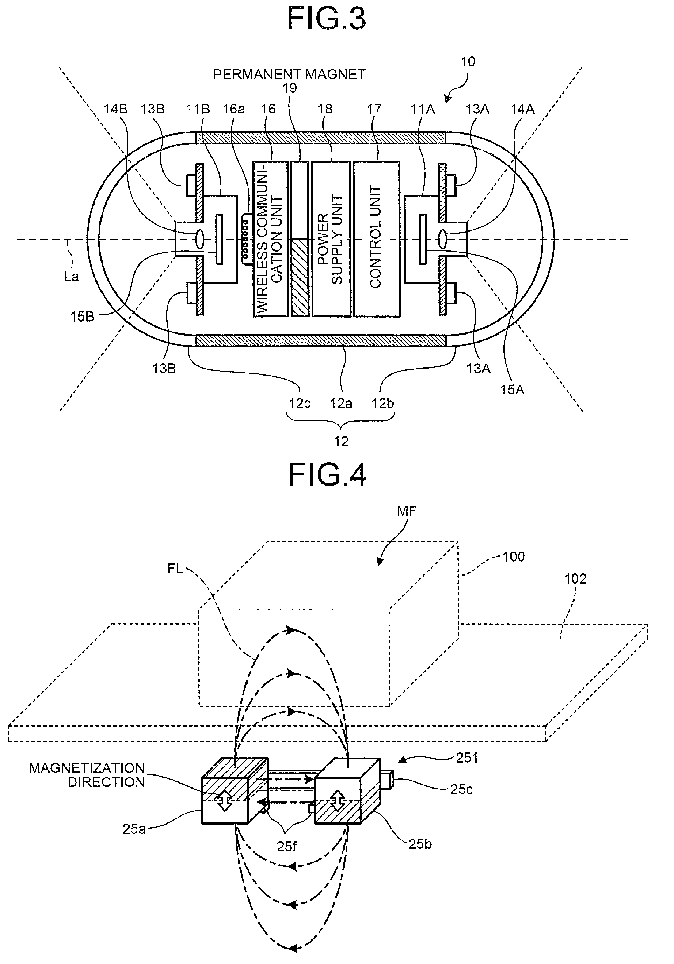

FIG. 3 is a cross sectional schematic diagram illustrating an example of an internal structure of the capsule-type endoscope 10. As illustrated in FIG. 3, the capsule-type endoscope 10 includes a capsule-shaped casing 12 which is an exterior formed to be of the size so that it can be easily introduced into the inside of an internal organ of the subject 101 and image-capturing units 11A, 11B generating image information by capturing images of the subject in the image-capturing directions which are different from each other. Further, the capsule-type endoscope 10 includes a wireless communication unit 16 for wirelessly transmitting, to the outside, image information generated by the image-capturing units 11A, 11B, a control unit 17 for controlling each constituent unit of the capsule-type endoscope 10, and a power supply unit 18 for providing electric power to each constituent unit of the capsule-type endoscope 10. Further, the capsule-type endoscope 10 includes a permanent magnet 19 enabling magnetic guidance with the guiding apparatus 20.

The capsule-shaped casing 12 is an exterior case formed to be of the size so that the capsule-shaped casing 12 can be introduced into the inside of the internal organ of the subject 101, and is made by shielding the opening ends at both sides of a cylindrical casing 12a with dome-shaped casings 12b, 12c. The dome-shaped casings 12b, 12c are dome-shaped optical members which are transparent to light of a predetermined wavelength band such as visible light. The cylindrical casing 12a is a colored casing which is substantially opaque to the visible light. As illustrated in FIG. 3, the capsule-shaped casing 12 formed by the cylindrical casing 12a and the dome-shaped casings 12b, 12c include, therein in liquid-tight manner, the image-capturing units 11A, 11B, the wireless communication unit 16, the control unit 17, the power supply unit 18, and the permanent magnet 19.

The image-capturing unit 11A includes an illumination unit 13A such as LED, an optical system 14A such as a condenser lens, and an image-capturing device 15A such as a CMOS image sensor or a CCD. The illumination unit 13A emits illumination light such as white light to an image-capturing vision field of the image-capturing device 15A, and illuminates a subject within image-capturing vision field through the dome-shaped casing 12b. The optical system 14A condenses the reflected light from the image-capturing vision field onto the image-capturing surface of the image-capturing device 15A, and forms the subject image of the image-capturing vision field on the image-capturing surface of the image-capturing device 15A. The image-capturing device 15A receives the reflected light from the image-capturing vision field condensed on the image-capturing surface and performs photoelectric conversion processing on the light signal thus received, thus generating the subject image of the image-capturing vision field, more specifically, generating image information representing an in-vivo image of the subject 101.

Like the image-capturing unit 11A, the image-capturing unit 11B includes an illumination unit 13B such as LED, an optical system 14B such as a condenser lens, and an image-capturing device 15B such as a CMOS image sensor or CCD.

As illustrated in FIG. 3, in a case of a twin-lens type capsule medical device in which the capsule-type endoscope 10 captures images at the front and back sides in the direction of the longitudinal axis La, the image-capturing units 11A, 11B are arranged so that each optical axis is substantially parallel to or substantially the same as the longitudinal axis La which is the central axis in the longitudinal direction of the capsule-shaped casing 12 and the image-capturing vision fields face directions opposite to each other. More specifically, the image-capturing units 11A, 11B are implemented so that the image-capturing surfaces of the image-capturing devices 15A, 15B are perpendicular to the longitudinal axis La.

The wireless communication unit 16 includes an antenna 16a, and successively, wirelessly transmits the image information obtained with the image-capturing units 11A, 11B explained above to the outside via the antenna 16a. More specifically, the wireless communication unit 16 obtains, from the control unit 17, an image signal based on the image information generated by the image-capturing unit 11A or the image-capturing unit 11B, and performs modulation processing and the like on the image signal, thus generating a wireless signal. The wireless communication unit 16 transmits this wireless signal to a reception unit 21, which is provided outside, via the antenna 16a.

The control unit 17 controls each operation of the image-capturing units 11A, 11B and the wireless communication unit 16, and controls input/output of signals between the constituent units. More specifically, the control unit 17 causes the image-capturing device 15A to capture an image of a subject in the image-capturing vision field which is illuminated by the illumination unit 13A, and causes the image-capturing device 15B to capture an image of a subject in the image-capturing vision field which is illuminated by the illumination unit 13B. The control unit 17 includes a signal processing function for generating an image signal. The control unit 17 obtains the image information from the image-capturing devices 15A, 15B, and on every such occasion, the control unit 17 performs the predetermined signal processing on the image information, and generates an image signal including image data. Further, the control unit 17 controls the wireless communication unit 16 so as to successively, wirelessly transmit the image signal to the outside in chronological order.

The power supply unit 18 is a battery unit such as a button-shaped battery or a capacitor, and includes a switch unit such as a magnetic switch and an optical switch. The power supply unit 18 switches the ON/OFF state of the power supply by the magnetic field that is applied from the outside, and when the ON/OFF state of the power supply is in the ON state, the power supply unit 18 provides, as necessary, the electric power of the battery unit to each constituent unit of the capsule-type endoscope 10 (image-capturing units 11A, 11B, wireless communication unit 16, and control unit 17). When the ON/OFF state of the power supply is in the OFF state, the power supply unit 18 stops the electric power provided to each constituent unit of the capsule-type endoscope 10.

The permanent magnet 19 is to enable magnetic guidance of the capsule-type endoscope 10 using the magnetic field MF generated by the guiding apparatus 20, and is arranged, in a fixed manner, inside of the capsule-shaped casing 12 so that the magnetization direction is inclined with respect to the longitudinal axis La. More specifically, the permanent magnet 19 is arranged so that the magnetization direction is perpendicular to the longitudinal axis La. The permanent magnet 19 operates so as to follow the magnetic field applied from the outside, and as a result, this achieves the magnetic guidance of the capsule-type endoscope 10 by the guiding apparatus 20.

As illustrated in FIG. 1, the guiding apparatus 20 includes reception unit 21 which wirelessly communicates with the capsule-type endoscope 10 and receives a wireless signal including image information obtained by the capsule-type endoscope 10, a position detection unit 22 which detects the position of the capsule-type endoscope 10 within the subject 101 on the basis of the wireless signal received from the capsule-type endoscope 10, a display unit 23a which obtains image information from the wireless signal received by the reception unit 21 and displays, on a screen, the in-vivo image and various kinds of information by performing predetermined signal processing on the image information, a notifying unit 23b which gives notification to the user by using visual information or audio information, an operation input unit 24 which receives input of information and the like for commanding various kinds of operations with the guiding system 1, a guiding-magnetic field generation unit 25 which generates magnetic field for magnetically guiding the capsule-type endoscope 10, a magnetic field state identifying unit 26 which identifies the state of the magnetic field generated by the guiding-magnetic field generation unit 25, a control unit 27 which controls each unit explained above, and a storage unit 28 which stores image information captured by the capsule-type endoscope 10.

The reception unit 21 includes a plurality of antennas 21a, and successively receives wireless signals from the capsule-type endoscope 10 via the plurality of antennas 21a. The reception unit 21 selects an antenna of which reception electric field strength is the highest from among the plurality of antennas 21a, and performs demodulation processing and the like on the wireless signal from the capsule-type endoscope 10 received via the selected antenna. Accordingly, the reception unit 21 extracts image information (image data) about the subject 101 from the wireless signal. The reception unit 21 outputs the image signal including the extracted image data to the display unit 23a.

The position detection unit 22 performs calculation for estimating the position of the capsule-type endoscope 10 in the subject 101 on the basis of the signal strength of the wireless signal received by the reception unit 21.

The display unit 23a includes various kinds of displays such as a liquid crystal display, and generates and displays, on the display, a screen including an in-vivo image based on the image data which are input from the reception unit 21 and various other kinds of information. More specifically, for example, the display unit 23a successively displays, on the screen, an in-vivo image group of the subject 101 captured by the capsule-type endoscope 10, and displays information about guiding operation and information about the position and the posture of the capsule-type endoscope 10. At this occasion, the display unit 23a may display the position and the posture of the capsule-type endoscope 10 which are estimated from the magnetic field generated by the guiding apparatus 20, and on the basis of the position detection result of the position detection unit 22, the display unit 23a may display, on the screen, the position within the subject 101 corresponding to the in-vivo image which is displayed. For example, the display unit 23a displays a reduced image of an in-vivo image selected according to the control of the control unit 27, and patient information, inspection information, and the like of the subject 101. Further, in accordance with the control of the control unit 27, the display unit 23a displays, on the screen, a warning given to a user, and information about the state of the magnetic field MF generated by the guiding-magnetic field generation unit 25 (for example, whether or not the magnetic field MF is generated in the effective magnetic field region 100).

The notifying unit 23b includes an illumination device such as LED, an audio device such as a buzzer, and a driving circuit for operating under the control of the control unit 27 in order to control these devices. The notifying unit 23b notifies warning to the user using blinking of illumination, buzzer sound, and the like, or notifies the user of the state of the magnetic field MF generated by the guiding-magnetic field generation unit 25 using the illumination in a predetermined color.

The operation input unit 24 is achieved with an input device such as an operation board and a keyboard having a joy stick, various kinds of button, and various kinds of switch, and receives input of various kinds of information such as guiding instruction information for magnetically guiding the capsule-type endoscope 10 and setting information for setting a predetermined mode with the guiding apparatus 20. The guiding instruction information is information for controlling the position and the posture of the capsule-type endoscope 10 which is magnetic guidance operation target, and more specifically, the guiding instruction information includes operation for changing the position of the capsule-type endoscope 10, information about operation for changing inclination angle (the angle with respect to the vertical axis) of the capsule-type endoscope 10, information about operation for changing the azimuth (the angle about the vertical axis) of the capsule-type endoscope 10. In the explanation below, the azimuth of the vision field will be simply referred to as azimuth. The operation input unit 24 inputs the received information into the control unit 27.

As illustrated in FIG. 2, the guiding-magnetic field generation unit 25 is provided at the lower portion of the bed 102 (the inner side of the leg portion of the bed 102), and generates, in the effective magnetic field region 100, the magnetic field MF for changing the position, the inclination angle, and the azimuth of the capsule-type endoscope 10 introduced into the subject 101. It should be noted that, in order to alleviate leakage of the magnetic field MF to the space other than the effective magnetic field region 100 (for example, the side surface direction of the leg portion of the bed 102), the leg portion of the bed 102 is preferably formed with ferromagnetic material such as an steel plate.

The guiding-magnetic field generation unit 25 includes a magnetic field generation unit 251 including a pair of permanent magnets 25a, 25b housed in cases 25d, 25e, respectively, a magnetic field state change driving unit 252 for changing the state of the magnetic field MF generated by the magnetic field generation unit 251, and also includes a flat plane position change unit 253, a vertical position change unit 254, an elevation angle change unit 255, and a traverse angle change unit 256, which serve as mechanisms for translationally moving the magnetic field generation unit 251 and rotating the magnetic field generation unit 251.

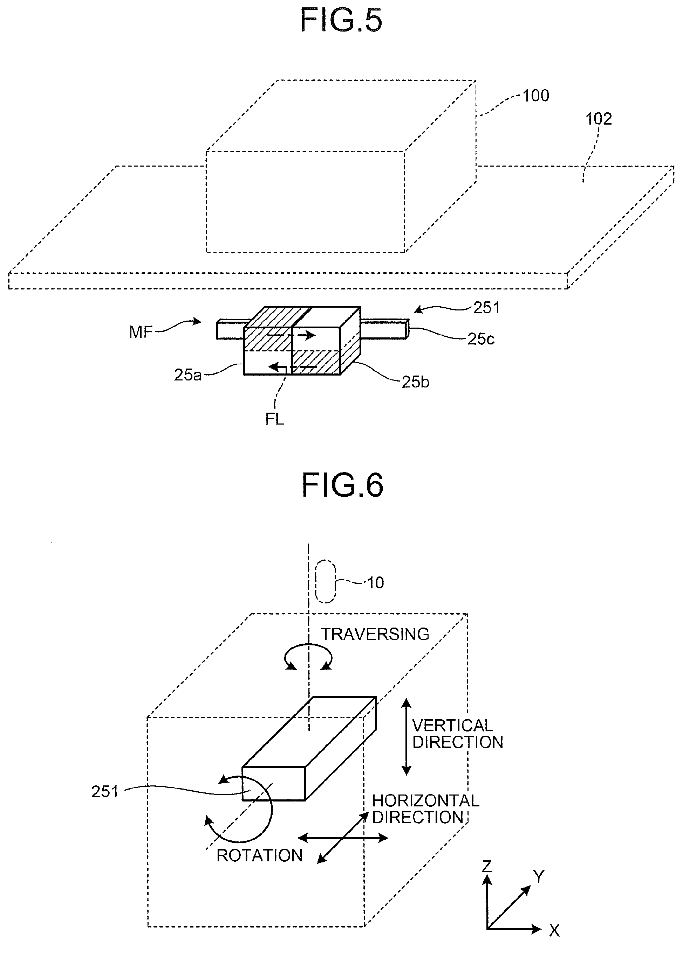

FIGS. 4 and 5 are schematic views illustrating configuration and operation principle of the magnetic field generation unit 251. As illustrated in FIGS. 4 and 5, for example, the permanent magnets 25a, 25b have rectangular parallelepiped shapes, and are arranged such that the surface perpendicular to the magnetization direction of each of them faces the bed 102. The permanent magnets 25a, 25b are arranged in such state that the magnetization directions thereof are parallel to each other and are opposite to each other. It should be noted that in FIGS. 4 and 5, the cases 25d, 25e are not shown.

The permanent magnets 25a, 25b are supported so that the relation positional relationship of each of them can be changed using a support member 25c by means of the cases 25d, 25e. More specifically, the support member 25c supports the permanent magnets 25a, 25b in such a manner that at least one of them can slide along the support member 25c. The support member 25c is provided with a stopper 25f engaging with the cases 25d, 25e or the permanent magnets 25a, 25b to prevent the permanent magnets 25a, 25b from moving in order to prevent the permanent magnets 25a, 25b from moving closer to each other due to magnetic attraction. With this stopper 25f, in normal circumstances, the permanent magnets 25a, 25b are in such state that the positions thereof are fixed with respect to the support member 25c. The stopper 25f is cancelled at a timing when the magnetic field state change driving unit 252 operates.

The magnetic field state change driving unit 252 changes the relative positional relationship of the permanent magnets 25a, 25b in order to change the state of the magnetic field MF generated by the magnetic field generation unit 251. More specifically, when the magnetic field MF is to be generated in the effective magnetic field region 100, the magnetic field state change driving unit 252 moves the permanent magnets 25a, 25b away from each other as illustrated in FIG. 4. Accordingly, magnetic force lines FL form open magnetic circuit extending to the peripheral region of the permanent magnet 25a and the permanent magnet 25b including the effective magnetic field region 100, and the magnetic field MF is generated in the effective magnetic field region 100. Hereinafter, this state will be referred to as the magnetic field generation state.

On the other hand, when the magnetic field MF is not to be generated in the effective magnetic field region 100, the magnetic field state change driving unit 252 moves the permanent magnets 25a, 25b close to each other as illustrated in FIG. 5. At this occasion, the permanent magnets 25a, 25b may be brought into direct contact with each other, or may be arranged with a slight gap maintained therebetween. Accordingly, the magnetic force lines FL form closed magnetic circuit which stays within the permanent magnets 25a, 25b and the close proximity thereto, and the magnetic field MF can be shielded from the effective magnetic field region 100. Hereinafter, this state will be referred to as a magnetic field shielded state. The magnetic field state change driving unit 252 is a switching unit for switching between the magnetic field generation state and the magnetic field shielded state.

While the relative positional relationship of the permanent magnets 25a, 25b arranged within the magnetic field generation unit 251 is maintained, the flat plane position change unit 253, the vertical position change unit 254, the elevation angle change unit 255, and the traverse angle change unit 256 translationally moves or rotates the magnetic field generation unit 251 as illustrated in FIG. 6. Accordingly, the distribution of the magnetic field MF within the effective magnetic field region is changed, whereby the position, the inclination angle, and the azimuth of the capsule-type endoscope 10 in the effective magnetic field region 100 are controlled. It should be noted that, in this first embodiment, the state in which the magnetization direction (see FIG. 4) of the permanent magnets 25a, 25b within the magnetic field generation unit 251 is in the vertical direction is defined as an initial state. In the initial state, the upper surface of the magnetic field generation unit 251 is parallel to the horizontal plane.

The flat plane position change unit 253 and the vertical position change unit 254 are achieved with a translational motion driving mechanism including, for example, a lifter and a slider. The flat plane position change unit 253 translationally moves the magnetic field generation unit 251 within the horizontal plane. The vertical position change unit 254 translationally moves the magnetic field generation unit 251 in the vertical direction.

The elevation angle change unit 255 rotates the magnetic field generation unit 251 with respect to the axis in the horizontal direction, and changes the angle of the upper surface of the magnetic field generation unit 251 with respect to the horizontal plane. Hereinafter, the angle formed by the upper surface of the magnetic field generation unit 251 and the horizontal plane will be referred to as an elevation angle θ.

The traverse angle change unit 256 rotates the magnetic field generation unit 251 with respect to the axis in the vertical direction passing through the center of the magnetic field generation unit 251. Hereinafter, the angle by which the magnetic field generation unit 251 rotates with respect to the initial state will be referred to as a traverse angle φ.

The azimuth and the inclination angle of the capsule-type endoscope 10 restrained by the magnetic field MF generated by the magnetic field generation unit 251 can be changed by causing the elevation angle change unit 255 to rotate the magnetic field generation unit 251 in such state that the traverse angle change unit 256 rotates the magnetic field generation unit 251 by the traverse angle φ.

The magnetic field state identifying unit 26 determines whether the guiding apparatus 20 is in the magnetic field generation state or the guiding apparatus 20 is in the magnetic field shielded state. The magnetic field state identifying unit 26 is achieved with, for example, a magnetic sensor provided in the effective magnetic field region 100 (for example, inside of the bed 102). When the output value of the magnetic sensor is equal to or more than a predetermined threshold value, the magnetic field state identifying unit 26 outputs an identification signal indicating that the guiding apparatus 20 is in the magnetic field generation state. On the other hand, when the output value of the magnetic sensor is less than the predetermined threshold value, the magnetic field state identifying unit 26 outputs an identification signal indicating that the guiding apparatus 20 is in the magnetic field shielded state.

The control unit 27 controls operation of the flat plane position change unit 253, the vertical position change unit 254, the elevation angle change unit 255, and the traverse angle change unit 256, on the basis of the guiding instruction information which is input from the detection result and operation input unit 24 of the position detection unit 22, thus realizing the position, the inclination angle, and the azimuth of the capsule-type endoscope 10 which are desired by the user. The control unit 27 controls the magnetic field state change driving unit 252 in accordance with the operation signal which is input from the operation input unit 24, thus changing the guiding apparatus 20 into a state according to the status of the capsule-type endoscope inspection (the magnetic field generation state or the magnetic field shielded state).

The storage unit 28 is achieved with a storage medium for saving information to a flash memory, a hard disk, or the like in a rewritable manner. The storage unit 28 stores not only image data of the in-vivo image group of the subject 101 captured by the capsule-type endoscope 10 but also information such as various kinds of programs and various kinds of parameters with which the control unit 27 controls each unit of the guiding apparatus 20.

Subsequently, the operation of the guiding system 1 as shown in FIG. 1 will be explained.

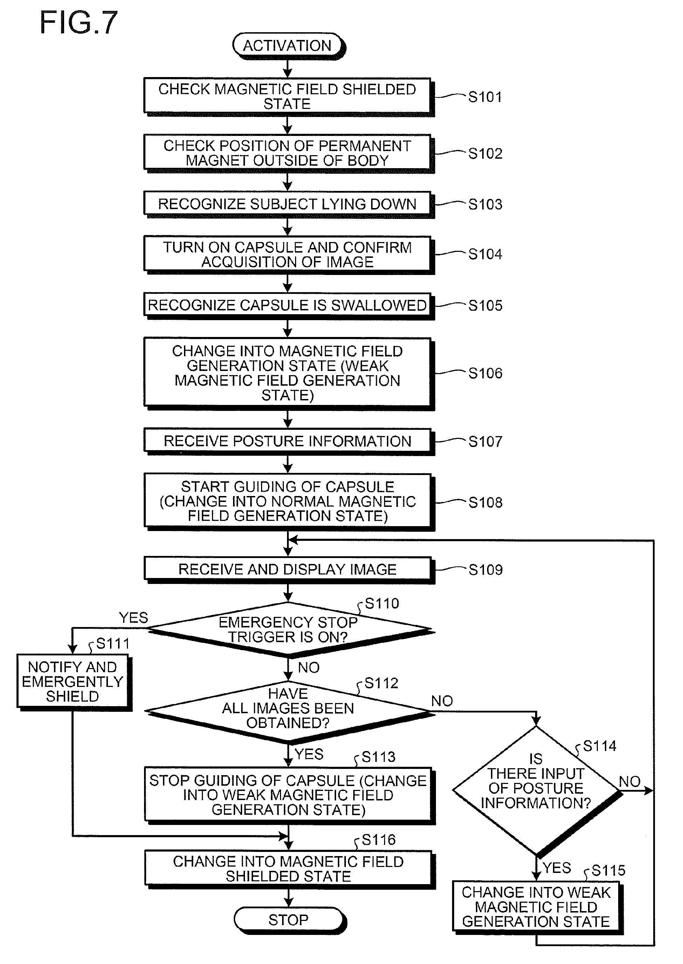

FIG. 7 is a flowchart illustrating operation of the guiding system 1. When the capsule medical device guiding system 1 is activated, first, in step S101, the control unit 27 of the guiding apparatus 20 checks, from the identification signal which is output by the magnetic field state identifying unit 26, whether the guiding apparatus 20 is in the magnetic field shielded state (see FIG. 5).

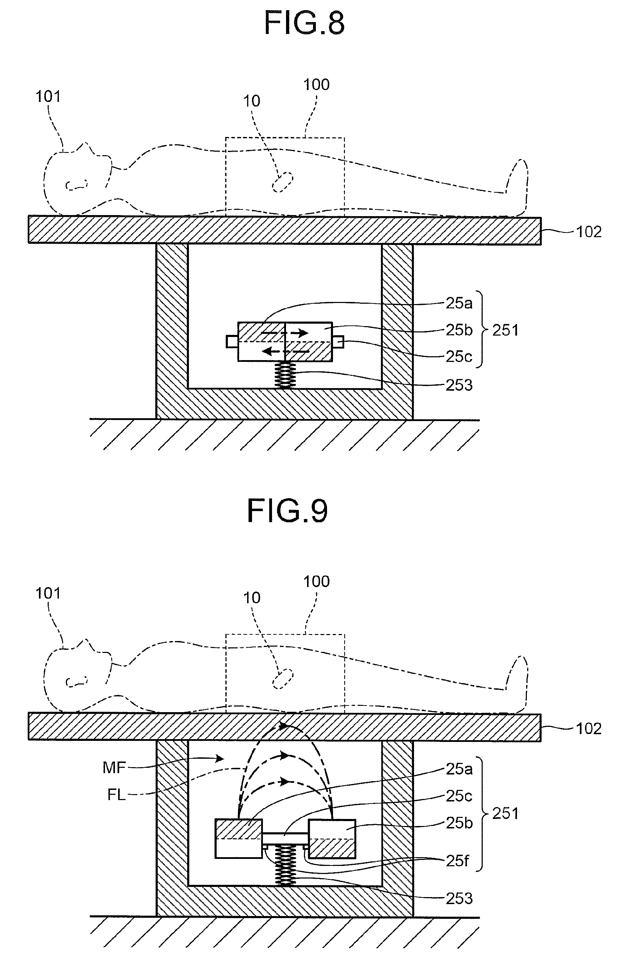

In step S102 subsequent thereto, the control unit 27 checks whether the magnetic field generation unit 251 is arranged at the position (initial position) at which the strength of the magnetic field in the effective magnetic field region 100 is the least. For example, as illustrated in FIG. 8, this initial position is the lowest position where the magnetic field generation unit 251 can be arranged within the leg portion of the bed 102 (more specifically, the initial position is a position farthest from the effective magnetic field region 100). It should be noted that, when the magnetic field generation unit 251 is not at the initial position, the control unit 27 controls the flat plane position change unit 253 and the vertical position change unit 254, and moves the magnetic field generation unit 251 to the initial position. At this occasion, the control unit 27 controls the elevation angle change unit 255 and the traverse angle change unit 256 to return the elevation angle θ and the traverse angle φ of the magnetic field generation unit 251 back to the initial state.

In step S103, when a signal indicating that the subject 101 lies on the bed 102 is input from the operation input unit 24, the control unit 27 recognizes that the subject 101 lies on the bed 102. This signal indicating that the subject 101 lies on the bed 102 may be input in response to a predetermined user operation (for example, by pressing a bed lie-down confirmation button). Alternatively, this signal indicating that the subject 101 lies on the bed 102 may be input when the bed 102 is in a predetermined state (for example, when a pressure sensor is provided on a subject lie-down surface of the bed 102, and the output value of this pressure sensor is more than a predetermined threshold value).

In step S104, when the power supply of the capsule-type endoscope 10 is turned ON, the guiding apparatus 20 receives a wireless signal transmitted from the capsule-type endoscope 10, and confirms that the image captured by the capsule-type endoscope 10 can be obtained. In this case, the capsule-type endoscope 10 is turned ON when the magnetic switch or the optical switch of the power supply unit 18 provided in the capsule-type endoscope 10 is turned ON. More specifically, the magnetism or the light is applied from the outside in order to activate the magnetic switch or the optical switch.

In step S105, when a signal indicating that the subject 101 swallows the capsule-type endoscope 10 is input from the operation input unit 24, the control unit 27 recognizes that the capsule-type endoscope 10 is swallowed by the subject 101. This signal indicating that the capsule-type endoscope 10 is swallowed may be input in response to a predetermined user operation (for example, by pressing a capsule swallowing confirmation button). Alternatively, the signal may be input when image data transmitted from the capsule-type endoscope 10 attain a predetermined state (for example, the color feature amount of the image attains a predetermined state indicating in-vivo state).

In step S106, when a signal indicating that inspection is started is input from the operation input unit 24, the control unit 27 controls each unit so as to change the guiding apparatus 20 into the magnetic field generation state. This signal indicating that the inspection is started may be input in response to, for example, an action of pressing a predetermined button of the operation input unit 24 (for example, inspection start button) and operation with a joy stick and the like.

Accordingly, as illustrated in FIG. 9, the magnetic field state change driving unit 252 slides any one of the permanent magnet 25a and the permanent magnet 25b with respect to the other of the permanent magnet 25a and the permanent magnet 25b so as to move the permanent magnet 25a and the permanent magnet 25b away from each other. As a result, the guiding apparatus 20 is in the magnetic field generation state. At this occasion, the magnetic field generation unit 251 is in the initial position (see step S102), and therefore, the strength of the magnetic field MF generated in the effective magnetic field region 100 becomes the least. Accordingly, this can prevent strong magnetic field from being rapidly applied to the capsule-type endoscope 10. Hereinafter, the magnetic field generation state in which the magnetic field generation unit 251 is at the initial position (state as illustrated in FIG. 9) will be referred to as weak magnetic field generation state.

In step S107, the control unit 27 receives information which is input from the operation input unit 24 (for example, posture information about the subject 101 such as left-side recumbent position), and displays the received information on the display apparatus 23a. At this occasion, the control unit 27 may obtain the relative coordinate system within the subject 101 on the basis of the absolute coordinate system of the capsule-type endoscope 10 (the coordinate system based on the gravity direction) and the information, and on the basis of the relative coordinate system, the control unit 27 may perform calculation processing such as estimation of the observation direction and the observation portion within the subject 101.

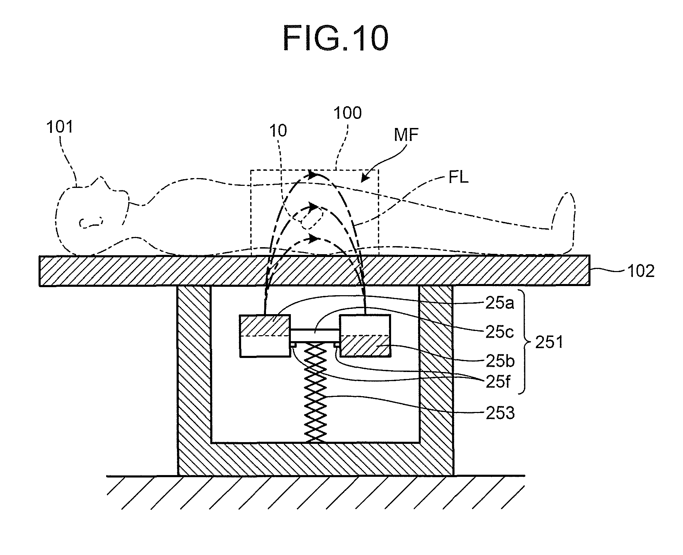

In step S108, each unit of the guiding-magnetic field generation unit 25 starts guiding of the capsule-type endoscope 10, and in accordance with the guiding instruction information which is input from the operation input unit 24, so that the position, the elevation angle θ, and the traverse angle φ of the magnetic field generation unit 251 are controlled. At this occasion, as illustrated in FIG. 10, the magnetic field generation unit 251 is moved to a position above the initial position, so that, in the effective magnetic field region 100, stronger magnetic field MF is formed. Accordingly, the guiding apparatus 20 changes from the weak magnetic field generation state to the normal magnetic field generation state.

In step S109, the control unit 27 causes the reception unit 21 to successively receive the wireless signal transmitted from the capsule-type endoscope 10, and causes the display unit 23a to display the image in the subject 101. While the user looks at the image, the user operates the operation input unit 24, so that the capsule-type endoscope 10 can be guided to the position, the inclination angle, and the azimuth desired by the user.

It should be noted that, as necessary the user may change the posture of the subject 101. In this case, the signal indicating that the posture of the subject 101 has been changed can be input into the guiding apparatus 20 by performing predetermined operation with the operation input unit 24 such as pressing down of the operation button and input of the posture information using a keyboard and the like. When a signal indicating that the posture of the subject 101 has been changed from the operation input unit 24 is input, the control unit 27 once returns the magnetic field generation unit 251 back to the initial position, so that it is in the weak magnetic field generation state. Accordingly, this can prevent strong magnetic field from being rapidly applied to the capsule-type endoscope 10 after the posture change.

In step S110, the control unit 27 determines whether an emergency stop trigger has been turned ON or not. In this case, the emergency stop trigger may be an emergency stop signal which is input from the operation input unit 24 in response to pressing of, for example, a predetermined button (emergency stop button) or may be a signal indicating that the subject 101 lying down who is once recognized in step S103 is no longer recognized (for example, decrease in the output value of a pressure sensor provided on the subject lie-down surface). In addition, detection due to vibration such as earthquake and the like and sudden decrease in the voltage in the guiding apparatus 20 may be adopted as the emergency stop trigger.

When the emergency stop trigger is turned ON (step S110: Yes), the control unit 27 causes the display unit 23a and the notifying unit 23b to execute notification indicating that the emergency stop trigger is turned on, and controls the magnetic field state change driving unit 252 to change the guiding apparatus 20 into the magnetic field shielded state (emergency shielded) (step S111). More specifically, the notifying method may be displaying a warning based on visual information such as "the system will emergency stop" on the display unit 23a, or the notifying unit 23b may be caused to execute warning based on other visual information such as blinking of illumination, or warning based on audio information such as voice or warning sound. Alternatively, both of them may be executed. Thereafter, in the operation of the guiding system 1, step S116 is subsequently performed. On the other hand, when the emergency stop trigger is not turned on (step S110: No), in the operation of the guiding system 1, step S112 is subsequently performed.

In step S112, the control unit 27 determines whether a signal indicting that all the images have been obtained is input from the operation input unit 24. The signal indicating that all the images have been obtained may be input in response to a predetermined user operation (for example, pressing down of an image acquisition-completion button or a guiding finish button). Alternatively, the signal may be input when a predetermined number of images have been received from the capsule-type endoscope 10 or when a predetermined period of time passes since the power supply of the capsule-type endoscope 10 is turned on.

When all the images have been obtained (step S112: Yes), the control unit 27 stops operation of the guiding-magnetic field generation unit 25 and stops guiding of the capsule-type endoscope 10, and returns the magnetic field generation unit 251 back to the initial position and changes the guiding apparatus 20 into the weak magnetic field generation state (see step S113, FIG. 9).

On the other hand, when all the images have not yet been obtained (step S112: No), the control unit 27 determines whether new posture information is input from the operation input unit 24 (step S114). When new posture information is input (step S114: Yes), the control unit 27 returns the magnetic field generation unit 251 back to the initial position and changes the guiding apparatus 20 into the weak magnetic field generation state (step S115). Thereafter, in the operation of the guiding system 1, step S109 is subsequently performed. On the other hand, when new posture information is not input (step S114: No), step S109 is subsequently performed in the operation of the guiding system 1.

In step S116, the control unit 27 controls the magnetic field state change driving unit 252 and changes the guiding apparatus 20 into the magnetic field shielded state (see FIG. 8). Accordingly, the operation of the guiding system 1 is terminated. Thereafter, the user drops the subject 101 from the bed 102.

As explained above, according to the first embodiment, in the guiding apparatus 20 which uses the permanent magnets 25a, 25b to generate the magnetic field MF for guiding the capsule-type endoscope 10, the positional relationship of the permanent magnets 25a, 25b is changed from the spaced apart state to the close proximity state, so that the guiding apparatus 20 can be changed from the magnetic field generation state to the magnetic field shielded state. Therefore, this achieves the small guiding apparatus 20 that can shielded the magnetic field when desired.

In addition, according to the first embodiment, the guiding-magnetic field generation unit 25 for guiding the capsule-type endoscope 10 is arranged below the bed 102 on which the subject 101 lies down, and therefore, the size of the entire capsule medical device guiding system can be further reduced.

In addition, according to the first embodiment, according to the status of the capsule-type endoscope inspection, the state of the magnetic field of the guiding apparatus 20 is changed with easy operation, and therefore, the inspection can be performed in a safe manner. For example, after the inspection is started, the guiding apparatus 20 is changed into the magnetic field generation state, and therefore, before and after the start of the inspection, the risk of attracting an unintended metal member with the magnetic field can be alleviated. In addition, for example, immediately after the guiding apparatus 20 is changed into the magnetic field generation state, the guiding apparatus 20 is changed into the state in which the strength of the magnetic field is the least, and therefore, the risk of abruptly applying strong magnetic field to the subject 101 can be avoided.

In addition, according to the first embodiment, when the inspection is started, or when the posture of the subject 101 is changed after the inspection was started, the magnetic field generation unit 251 is returned back to the initial position, and the guiding apparatus 20 is changed into the weak magnetic field generation state, and therefore, this can prevent strong magnetic field from being applied to the capsule-type endoscope 10, and prevent the capsule-type endoscope 10 from moving to the position which is not expected by the user..

It should be noted that, according to the first embodiment explained above, during transition from the magnetic field shielded state to the magnetic field generation state, it is necessary to use force for moving the permanent magnets 25a, 25b, and therefore, the load of the force can be reduced by making the cases 25d, 25e using a material of which frictional coefficient is small and improving the moving mechanism.

In addition, according to the first embodiment a multi-lens capsule having the image-capturing units 11A, 11B provided at both ends of the capsule-type endoscope 10 is used. Alternatively, a single lens capsule having an image-capturing unit provided at any one of the ends of the capsule-type endoscope may be used.

In addition, according to the first embodiment explained above, the permanent magnet 19 is arranged so that the magnetization direction is perpendicular to the longitudinal axis La of the capsule-type endoscope 10. Alternatively, the permanent magnet 19 may be arranged so that the magnetization direction matches the direction of the longitudinal axis La.

In addition, according to the first embodiment explained above, the guiding-magnetic field generation unit 25 is used to generate the magnetic field MF for guiding the capsule-type endoscope 10 introduced into the subject 101. Alternatively, magnetic fields giving various kinds of effects on the capsule-type endoscope 10 may be generated. For example, the magnetic switch may be incorporated into the capsule-type endoscope 10, and the magnetic switch may be turned ON/OFF by the guiding-magnetic field generation unit 25 in a remote control manner.

### (Modification 1-1)

In modification 1-1 of the first embodiment, the magnetic field state identifying unit 26 may be achieved with a position sensor for detecting the position of the permanent magnets 25a, 25b. For example, when the interval between the permanent magnet 25a and the permanent magnet 25b is equal to or more than a predetermined threshold value (the state as shown in FIG. 4), the magnetic field state identifying unit 26 outputs an identification signal indicating that the guiding apparatus 20 is in the magnetic field generation state. On the other hand, when the interval between the permanent magnet 25a and the permanent magnet 25b is less than the predetermined threshold value (the state as shown in FIG. 5), the magnetic field state identifying unit 26 outputs an identification signal indicating that the guiding apparatus 20 is in the magnetic field shielded state.

### (Modification 1-2)

The position detection of the capsule-type endoscope 10 within the subject 101 may be done by not only a method based on the strength of the wireless signal received from the capsule-type endoscope 10 as explained in the first embodiment but also various methods.

For example, a method for detecting the position of the capsule-type endoscope 10 on the basis of the acceleration applied to the capsule-type endoscope 10 may be employed. In this case, an acceleration sensor for detecting the acceleration applied to the capsule-type endoscope 10 in a three-dimensional manner is provided inside of the capsule-type endoscope 10, and the detection result given by the acceleration sensor is multiplexed with a wireless signal and is transmitted as necessary. On the basis of the detection result given by the acceleration sensor multiplexed with the wireless signal received, the guiding apparatus 20 adds the acceleration applied to the capsule-type endoscope 10 to derive the amount of relative change of the position of the capsule-type endoscope 10, and calculates the current position of the capsule-type endoscope 10 from this amount of change.

### (Modification 1-3)

In another position detection method of the capsule-type endoscope 10 in the subject 101, a method for detecting an alternate current magnetic field may also be used. In this case, an alternate current magnetic field generation unit for generating an alternate current magnetic field is provided inside of the capsule-type endoscope 10. On the other hand, at the guiding apparatus 20, a plurality of magnetic field sensors for detecting the alternate current magnetic field are provided.

The guiding apparatus 20 uses each of the plurality of magnetic field sensors arranged at a plurality of positions to detect the alternate current magnetic field generated by the capsule-type endoscope 10, and on the basis of the detection results, the guiding apparatus 20 continuously calculates at least one of the position, the azimuth, and the inclination angle of the capsule-type endoscope 10. At this occasion, the guiding apparatus 20 may control the guiding-magnetic field generated by itself on the basis of at least one of the position, the azimuth, and inclination angle of the capsule-type endoscope 10 calculated. The guiding apparatus 20 may determine whether the position of the capsule-type endoscope 10 is located within the measurement region in the subject 101 (a region of the magnetic field generated by the guiding-magnetic field generation unit 25), and may control the operation of the magnetic field state change driving unit 252 on the basis of the determination result. For example, when the capsule-type endoscope 10 is located in the measurement region in the subject 101, the control unit 27 controls the magnetic field state change driving unit 252, and changes the permanent magnets 25a, 25b into the spaced apart state so as to attain the magnetic field generation state. On the other hand, when the capsule-type endoscope 10 is located outside of the measurement region in the subject 101, the control unit 27 controls the magnetic field state change driving unit 252, and changes the permanent magnets 25a, 25b into the close proximity state so as to attain the magnetic field shielded state.

### (Modification 1-4)

Another method for detecting an alternate current magnetic field will be explained as a position detection method for detecting the capsule-type endoscope 10 in the subject 101. In this case, an LC circuit resonated by the alternate current magnetic field is provided inside of the capsule-type endoscope 10, and the guiding apparatus 20 is provided with an alternate current magnetic field generation apparatus and a plurality of magnetic field sensors for detecting an alternate current magnetic field.

The guiding apparatus 20 detects, in advance, a first alternate current magnetic field generated by an alternate current magnetic field generation apparatus in such state that the capsule-type endoscope 10 is not located in the measurement region in the subject 101 (a region of the magnetic field generated by the magnetic field generation unit 25). Then, when the capsule-type endoscope 10 is located in the measurement region in the subject 101, a second alternate current magnetic field including a resonant magnetic field generated by the LC circuit in the capsule-type endoscope 10 is detected, and the resonant magnetic field generated by the LC circuit in the capsule-type endoscope 10 is derived from the difference value of the detection value of the first alternate current magnetic field and the detection value of the second alternate current magnetic field. On the basis of the resonant magnetic field thus derived, the guiding apparatus 20 continuously calculates the position coordinate of the capsule-type endoscope 10 in the three-dimensional space.

### (Modification 1-5)

In modification 1-5 of the first embodiment, the magnetic field state identifying unit 26 may be constituted by a sensor for detecting the force between the permanent magnets 25a, 25b (the magnetic attraction or the magnetic repulsive force).

In this case, in the magnetic field generation state and the magnetic field shielded state, the direction and the strength of the magnetic force between the permanent magnets 25a, 25b is different. For example, as illustrated in FIG. 4, in a case where the magnetic force line FL is open magnetic circuit (more specifically, in a case of magnetic generation state), the magnetic repulsive force between the permanent magnets 25a, 25b is weak. On the contrary, as illustrated in FIG. 5, when the magnetic force line FL is closed magnetic circuit (more specifically in a case of magnetic shielded state), the magnetic attraction between the permanent magnets 25a, 25b is strong. Accordingly, any one of the permanent magnets 25a, 25b is provided with a pressure sensor for detecting the pressure applied to the permanent magnet, and by determining whether attraction force is exerted between the permanent magnets 25a, 25b from the detection value of the pressure sensor (the direction and the strength), whether the guiding apparatus is in the magnetic field generation state or in the magnetic field shielded state can be identified.

### (Second embodiment)

Subsequently, the second embodiment of the present invention will be explained.

In the first embodiment explained above, the interval of the permanent magnets 25a, 25b is changed, whereby the magnetic field generation state and the magnetic field shielded state are changed from one another. Alternatively, using various methods other than the above, these states can be changed from one another.

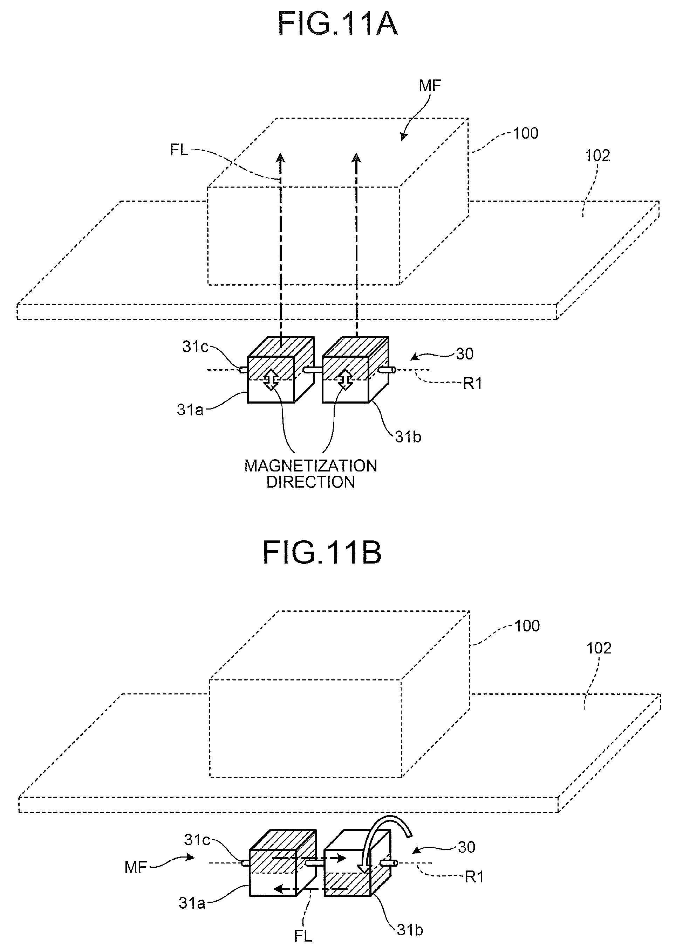

FIGS. 11A and 11B are schematic views illustrating a configuration of a magnetic field generation unit provided in a guiding apparatus according to the second embodiment. FIG. 11A illustrates the magnetic field generation state. FIG. 11B illustrates the magnetic field shielded state. As shown in FIGS. 11A and 11B, the guiding apparatus according to the second embodiment has a magnetic field generation unit 30 instead of the magnetic field generation unit 251 as shown in FIG. 2. The magnetic field generation unit 30 includes a pair of permanent magnets 31a, 31b which are arranged such that the surface perpendicular to the magnetization direction are arranged side by side to face the bed 102, and also includes a support member 31c for supporting the permanent magnets 31a, 31b so that the permanent magnets 31a, 31b can rotate in any one of the directions. It should be noted that the configuration of the guiding apparatus according to the second embodiment except the magnetic field generation unit 30 is the same as what is shown in FIGS. 2 and 4. In FIGS. 11A, 11B, cases for accommodating the permanent magnets 31a, 31b are not shown.

As shown in FIG. 11A, in the magnetic field generation state, the permanent magnets 31a, 31b are arranged in such state that the same poles as each of them at the surface parallel to the magnetization direction are arranged to face each other. At this occasion, the magnetic force lines extending from the permanent magnets 31a, 31b form open magnetic circuit in a direction substantially parallel to each magnetization direction. Accordingly, the magnetic field MF is generated in a range including the effective magnetic field region 100 on the bed 102.

At the transition from the magnetic field generation state to the magnetic field shielded state, the magnetic field state change driving unit 252 (see FIG. 2) rotates one of the permanent magnets 31a, 31b about an axis R1 passing through the permanent magnets 31a, 31b, and as shown in FIG. 11B, the poles different from each other at the surface parallel to the magnetization direction are arranged to face each other. At this occasion, the magnetic force line FL form closed magnetic circuit which stays within the permanent magnets 31a, 31b and the proximity thereto. Accordingly, in this state, the magnetic field MF is shielded from the effective magnetic field region 100.

### (Modification 2-1)

Like modification 1-5, modification 2-1 of the second embodiment is such that the magnetic field state identifying unit 26 may be constituted by a sensor for detecting the force between the permanent magnets 31a, 31b (the magnetic attraction or the magnetic repulsive force). In modification 2-1, whether the force exerted between the permanent magnets 31a, 31b is attracting force or repulsive force is determined from the detection value of the pressure sensor (the direction and the strength), so that whether the guiding apparatus is in the magnetic field generation state or in the magnetic field shielded state can be identified.

### (Third embodiment)

Subsequently, a third embodiment of the present invention will be explained.

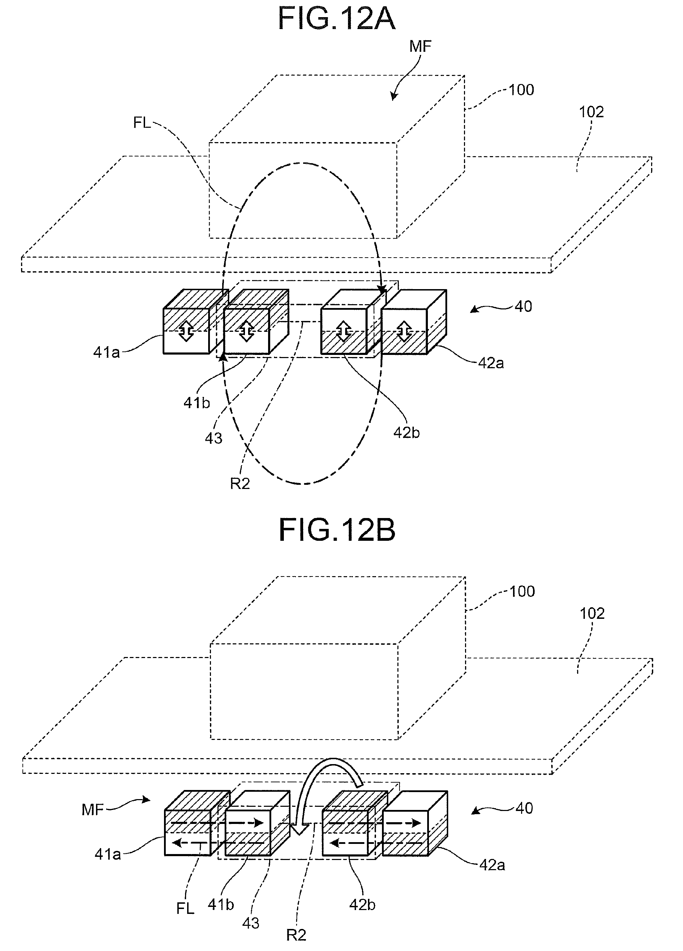

FIGS. 12A and 12B are schematic views illustrating configuration a magnetic field generation unit provided in a guiding apparatus according to the third embodiment. FIG. 12A illustrates a magnetic field generation state, and FIG. 12B illustrates a magnetic field shielded state. As shown in FIGS. 12A and 12B, the guiding apparatus according to the third embodiment has a magnetic field generation unit 40 instead of the magnetic field generation unit 251 as shown in FIG. 2. The magnetic field generation unit 40 includes two pairs of permanent magnets (41a, 41b) and (42a, 42b) which are arranged such that the surface perpendicular to the magnetization direction are arranged side by side to face the bed 102. The permanent magnets 41a, 42a are held such that the poles different from each other at the surface parallel to the magnetization direction are arranged to face each other, and the permanent magnets 41a, 42a are held at fixed positions by a support member, not shown. On the other hand, the permanent magnets 41b, 42b are held by a holding unit 43 in such state that the poles different from each other at the surface parallel to the magnetization direction are arranged to face each other. It should be noted that the configuration of the guiding apparatus according to the third embodiment except the magnetic field generation unit 40 is the same as what is shown in FIGS. 2 and 4. In FIGS. 12A, 12B, cases for accommodating the permanent magnets 41a, 41b are not shown.

As shown in FIG. 12A, in the magnetic field generation state, the permanent magnets included in each of the permanent magnet pairs (41a, 41b), (42a, 42b) are arranged such that the poles different from each other at the surface parallel to the magnetization direction are arranged to face each other. At this occasion, the magnetic force lines FL extending from the permanent magnets 41a, 41b form open magnetic circuit which makes a large curve about the space around the magnetic field generation unit 40 and converges to the permanent magnet 42a, 42b. Accordingly, the magnetic field MF is generated in a range including the effective magnetic field region 100.

During the transition from the magnetic field generation state to the magnetic field shielded state, the magnetic field state change driving unit 252 (see FIG. 2) rotates the holding unit 43 about an axis R2 passing through the permanent magnets 41b, 42b, so that the poles different from each other in the each of the permanent magnet pairs (41a, 41b), (42a, 42b) are arranged to face each other. At this occasion, the magnetic force lines FL form closed magnetic circuit staying within each of the permanent magnet pairs (41a, 41b), (42a, 42b) and the proximity thereto. Accordingly, in this state, the magnetic field MF is shielded from the effective magnetic field region 100.

It should be noted that, during the transition from the magnetic field generation state to the magnetic field shielded state, the relative relationship with respect to the permanent magnets 41b, 42b may be changed by rotating the permanent magnets 41a, 42a instead of rotating the permanent magnets 41b, 42b.

### (Modification 3-1)

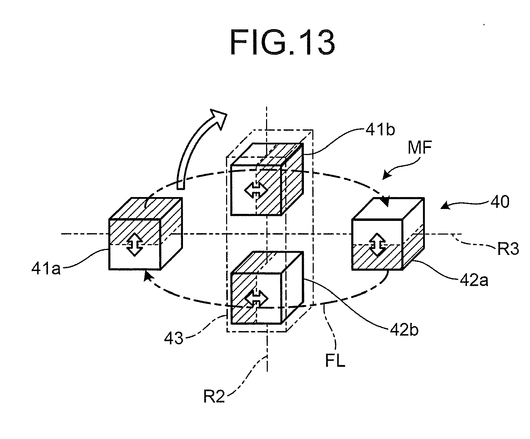

In a modification of the third embodiment, when the magnetic field generation unit 40 as shown in FIG. 12A is changed into the magnetic field shielded state, the holding unit 43 may be rotated so that the magnetization directions cross in each of the permanent magnet pairs (41a, 41b), (42a, 42b). For example, as illustrated in FIG. 13, when the holding unit 43 is rotated so that the axis R2 and the axis R3 passing through the permanent magnets 41a, 42a are perpendicular to each other, the magnetization directions are perpendicular in each of the permanent magnet pairs (41a, 41b), (42a, 42b), and the pole surface different from each other are in proximity to each other. In this case, the magnetic force lines FL form closed magnetic circuit which circulates the permanent magnet 41a, the permanent magnet 41b, the permanent magnet 42a, and then the permanent magnet 42b. Accordingly, in this state, the magnetic field MF is shielded from the effective magnetic field region 100 (see FIG. 12A).

### (Modification 3-2)

Like modification 2, another modification of the third embodiment may be configured such that the magnetic field state identifying unit 26 may be constituted by a sensor for detecting the force between the permanent magnets 41a, 41b or between the permanent magnets 42a, 42b (the magnetic attraction or the magnetic repulsive force).

### (Fourth embodiment)

Subsequently, a fourth embodiment of the present invention will be explained.

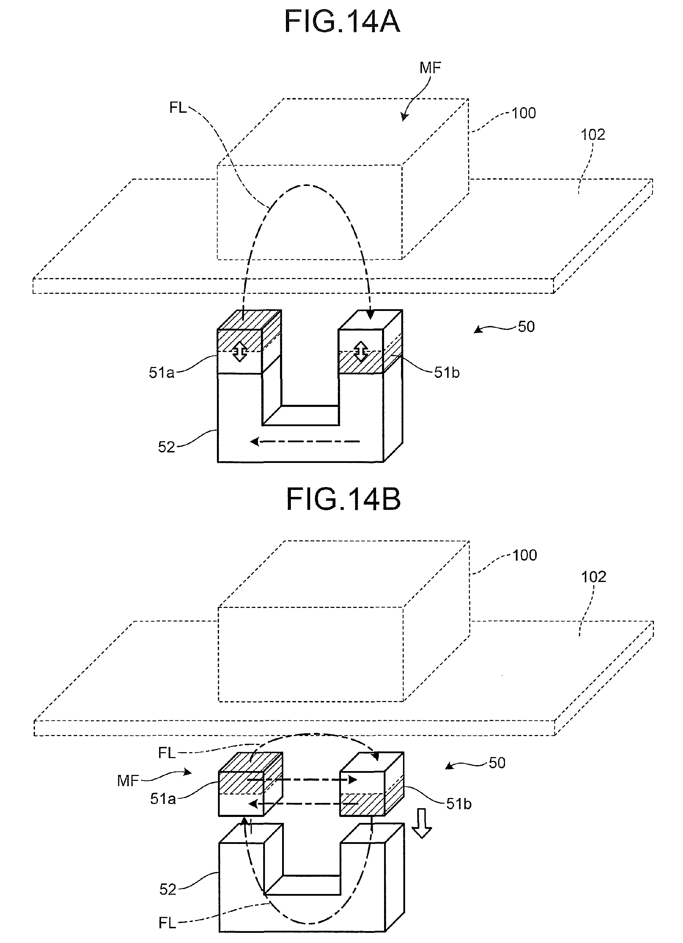

FIGS. 14A and 14B are schematic views illustrating configuration a magnetic field generation unit provided in a guiding apparatus according to the fourth embodiment. FIG. 14A illustrates a magnetic field generation state, and FIG. 14B illustrates a magnetic field shielded state. As shown in FIGS. 14A and 14B, the guiding apparatus according to the fourth embodiment has a magnetic field generation unit 50 instead of the magnetic field generation unit 251 as shown in FIG. 2. The magnetic field generation unit 50 includes a pair of permanent magnets 51a, 51b which are arranged to be spaced apart and the surface perpendicular to the magnetization direction are arranged to face the bed 102, and also includes a magnetic shield member 52 made of ferromagnetic body such as steel. The permanent magnets 51a, 52b are held such that the poles different from each other at the surface parallel to the magnetization direction are arranged to face each other, and the permanent magnets 51a, 51b are held by a support member, not shown. It should be noted that the configuration of the guiding apparatus according to the fourth embodiment except the magnetic field generation unit 50 is the same as what is shown in FIGS. 2 and 4. In FIGS. 14A, 14B, cases for accommodating the permanent magnets 51a, 51b are not shown.

As shown in FIG. 14A, in the magnetic field generation state, the magnetic shield member 52 is arranged to come into contact with the pole surface (the surface at the lower side in FIG. 14A) of the permanent magnets 51a, 52b opposite to the effective magnetic field region 100. At this occasion, the magnetic force lines FL extending from the permanent magnet 51a form open magnetic circuit which makes a large curve about the space above the magnetic field generation unit 50 and converges to the permanent magnet 51b. Accordingly, the magnetic field MF is generated in a range including the effective magnetic field region 100 on the bed 102.

During the transition from the magnetic field generation state to the magnetic field shielded state, the magnetic field state change driving unit 252 (see FIG. 2) moves the magnetic shield member 52 away from the pole surfaces of the permanent magnets 51a, 51b as shown in FIG. 14B. At this occasion, at the lower side of the figure of the permanent magnets 51a, 51b, the magnetic force lines FL extends from the permanent magnet 51b via the magnetic shield member 52 and converges to the permanent magnet 51a, and on the other hand, at the upper side of the figure of the permanent magnets 51a, 51b, the magnetic force lines FL stay in proximity to the permanent magnets 51a, 51b. Accordingly, in this state, the magnetic field MF is shielded from the effective magnetic field region 100.

It should be noted that, in the fourth embodiment, the same effects can be obtained even if a permanent magnet of which magnetization direction is specified is used instead of the magnetic shield member 52.

### (Fifth embodiment)

Subsequently, a fifth embodiment of the present invention will be explained.

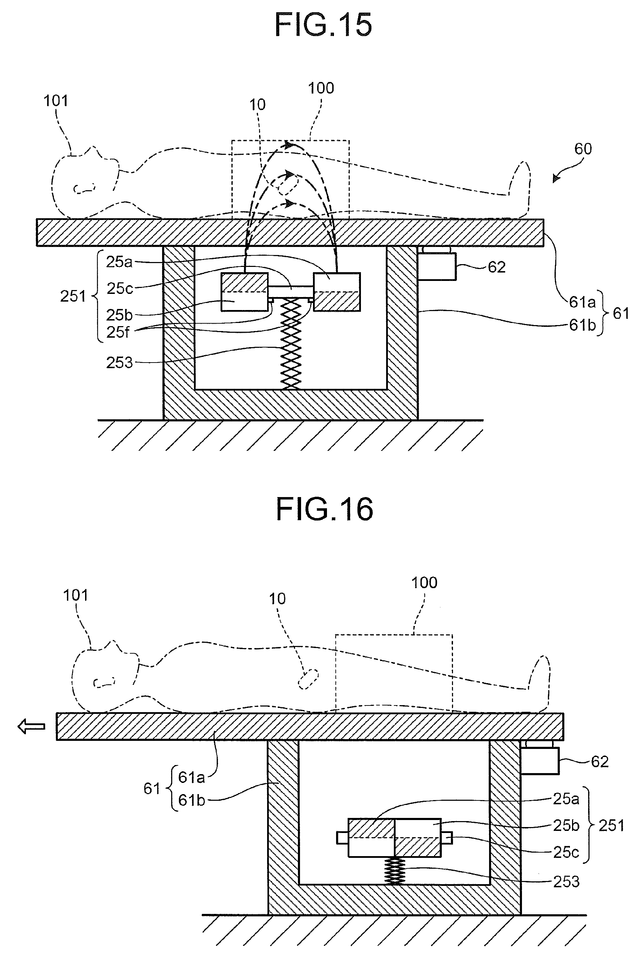

FIG. 15 is a schematic view illustrating configuration of a guiding apparatus according to the fifth embodiment. As illustrated in FIG. 15, a guiding apparatus 60 according to the fifth embodiment is based on the guiding apparatus 20 as shown in FIG. 2 and further includes a bed 61 in which a subject lie-down unit 61a is configured to be able to slide with respect to a leg unit 61b, and further includes a bed driving unit 62 for driving a subject lie-down unit 61a. The configuration of the guiding apparatus 60 except the bed 61 and the bed driving unit 62 is the same as the first embodiment.

On the lower surface of the subject lie-down unit 61a, a rail extending in the height direction of the subject 101 (the horizontal direction in FIG. 15) is provided, and with this rail, the subject lie-down unit 61a can slide with respect to the leg unit 61b in the height direction (see FIG. 16). The bed driving unit 62 operates under the control of the control unit 27, and moves the subject lie-down unit 61a in the height direction explained above.

When the guiding apparatus 60 is changed into the magnetic field shielded state, the control unit 27 cooperates with the magnetic field state change driving unit 252 to operate the bed driving unit 62, and as illustrated in FIG. 16, the inspection target portion of the subject 101 (for example, stomach portion) is moved out of the effective magnetic field region 100.

According to the fifth embodiment explained above, when the guiding apparatus 60 is changed into the magnetic field shielded state, the effect exerted on the capsule-type endoscope 10 by the magnetic field generated by the magnetic field generation unit 251 can be further reduced.

It should be noted that the configuration of the bed 61 and the bed driving unit 62 explained in the fifth embodiment may be provided in the guiding apparatus according to the second to fourth embodiments.

The embodiments explained above are merely examples for carrying out the present invention, and the present invention is not limited thereto. In the present invention, various inventions can be generated by combining, as necessary, a plurality of constituent elements disclosed in each embodiment and modification. The present invention can be changed in various manners in accordance with specifications and the like, and further, it is understood from the above description that various other embodiments can be made within the scope of the present invention.

### Reference Signs List

- 1: CAPSULE MEDICAL DEVICE GUIDING SYSTEM
- 10: CAPSULE-TYPE ENDOSCOPE
- 11A, 11B: IMAGE-CAPTURING UNIT
- 12: CAPSULE-SHAPED CASING
- 12a: CYLINDRICAL CASING
- 12b, 12c: DOME-SHAPED CASING
- 13A, 13B: ILLUMINATION UNIT
- 14A, 14B: OPTICAL SYSTEM
- 15A, 15B: IMAGE-CAPTURING DEVICE
- 16: WIRELESS COMMUNICATION UNIT
- 16a: ANTENNA
- 17: CONTROL UNIT
- 18: POWER SUPPLY UNIT
- 19: PERMANENT MAGNET
- 20, 60: GUIDING APPARATUS
- 21a: ANTENNA
- 21: RECEPTION UNIT
- 22: POSITION DETECTION UNIT
- 23a: DISPLAY UNIT
- 23b: NOTIFYING UNIT
- 24: OPERATION INPUT UNIT
- 25: GUIDING-MAGNETIC FIELD GENERATION UNIT
- 251, 30, 40, 50: MAGNETIC FIELD GENERATION UNIT
- 252: MAGNETIC FIELD STATE CHANGE DRIVING UNIT
- 253: FLAT PLANE POSITION CHANGE UNIT
- 254: VERTICAL POSITION CHANGE UNIT
- 255: ELEVATION ANGLE CHANGE UNIT
- 256: TRAVERSE ANGLE CHANGE UNIT
- 25a, 25b, 31a, 31b, 41a, 41b, 42a, 42b, 51a, 51b: PERMANENT MAGNET
- 25c, 31c: SUPPORT MEMBER
- 25d, 25e: CASE
- 25f: STOPPER
- 26: MAGNETIC FIELD STATE IDENTIFYING UNIT
- 27: CONTROL UNIT
- 28: STORAGE UNIT
- 43: HOLDING UNIT
- 52: MAGNETIC SHIELD MEMBER
- 61, 102: BED (SUBJECT BED)
- 61a: SUBJECT LIE-DOWN UNIT
- 61b: LEG UNIT
- 62: BED DRIVING UNIT
- 100: EFFECTIVE MAGNETIC FIELD REGION
- 101: SUBJECT

## Claims

1. A magnetic field generation apparatus for generating a guiding-magnetic field which is a magnetic field for magnetically guiding a target object, the magnetic field generation apparatus comprising:
a plurality of permanent magnets configured to generate a magnetic field; and
a switching unit configured to change relative relationship of the plurality of permanent magnets to switch between a first state in which the plurality of permanent magnets generate the guiding-magnetic field in a predetermined region and a second state in which the plurality of permanent magnets do not generate the guiding-magnetic field in the region.

2. The magnetic field generation apparatus according to claim 1, wherein, in the first state, the plurality of permanent magnets form open magnetic circuit, and
in the second state, the plurality of permanent magnets form closed magnetic circuit.

3. The magnetic field generation apparatus according to claim 1, wherein the plurality of permanent magnets are arranged such that magnetization directions are parallel to each other and different poles are arranged to face each other,
wherein, in the first state, the plurality of permanent magnets are spaced apart, and
in the second state, the plurality of permanent magnets are close to each other.

4. The magnetic field generation apparatus according to claim 1, wherein the plurality of permanent magnets are arranged such that magnetization directions are parallel to each other,
wherein, in the first state, the plurality of permanent magnets are arranged such that same poles are arranged to face each other, and
in the second state, the plurality of permanent magnets are arranged such that poles different from each other are arranged to face each other.

5. The magnetic field generation apparatus according to claim 4, wherein in the second state, the plurality of permanent magnets are configured such that pole surfaces different from each other are further brought in proximity to each other.

6. The magnetic field generation apparatus according to claim 1, wherein the plurality of permanent magnets arranged to be spaced apart from each other such that magnetization directions are parallel to each other and different poles are arranged to face each other, and
the magnetic field generation apparatus further comprises a magnetic shield member made of a ferromagnetic material,
wherein, in the first state, the magnetic shield member is in contract with the pole surfaces of the permanent magnets, and
in the second state, the magnetic shield member is spaced apart from the pole surface.

7. The magnetic field generation apparatus according to claim 1 further comprising:
a magnetic field state identifying unit configured to identify the first state and the second state; and
a notifying unit configured to notify an identifying result given by the magnetic field state identifying unit.

8. The magnetic field generation apparatus according to claim 7, wherein the magnetic field state identifying unit detects the magnetic field generated by the magnetic field generation apparatus.

9. The magnetic field generation apparatus according to claim 7, wherein the magnetic field state identifying unit detects force between the plurality of permanent magnets.

10. The magnetic field generation apparatus according to claim 7, wherein the notifying unit notifies the identifying result with visual information.

11. The magnetic field generation apparatus according to claim 7, wherein the notifying unit notifies the identifying result with audio information.

12. The magnetic field generation apparatus according to claim 1, wherein the target object is a capsule medical device including a magnet and introduced into a subject.

13. A capsule medical device guiding system comprising:
a magnetic field generation apparatus for generating a guiding-magnetic field which is a magnetic field for magnetically guiding a target object, wherein the magnetic field generation apparatus includes a plurality of permanent magnets configured to generate a magnetic field, and a switching unit configured to change relative relationship of the plurality of permanent magnets to switch between a first state in which the plurality of permanent magnets generate the guiding-magnetic field in a predetermined region and a second state in which the plurality of permanent magnets do not generate the guiding-magnetic field in the region; and
a capsule medical device including a magnet and introduced into a subject, wherein the capsule medical device is magnetically guided by the guiding-magnetic field in the predetermined region.

14. A capsule medical device guiding system according to claim 13 further comprising:
a magnetic field state identifying unit configured to identify the first state and the second state; and
a control unit configured to control operation of the switching unit in accordance with an identifying result given by the magnetic field state identifying unit and a status of inspection with the capsule medical device.
